(19) Europäisches Patentamt
European Patent Office
Office européen des brevets

(11) Numéro de publication : **0 382 636 B1**

# (12) FASCICULE DE BREVET EUROPEEN

(45) Date de publication du fascicule du brevet : **28.07.93 Bulletin 93/30**

(51) Int. Cl.$^5$ : **C07D 403/12,** C07D 239/42, A61K 31/505

(21) Numéro de dépôt : **90400336.5**

(22) Date de dépôt : **07.02.90**

## (54) Dérivés de pyrimidine, 2-[4-(alpha-heteroaryl-alpha aryl-(alpha-alkyl)-methoxy)-butyl)-1-piperazinyl], avec activité serotoninergique.

(30) Priorité : **09.02.89 FR 8901699**

(43) Date de publication de la demande : **16.08.90 Bulletin 90/33**

(45) Mention de la délivrance du brevet : **28.07.93 Bulletin 93/30**

(84) Etats contractants désignés : **AT BE CH DE DK FR GB GR IT LI LU NL SE**

(56) Documents cités :
**CHEMICAL ABSTRACTS, vol. 75, 1971, page 165, résumé no. 31808x, Columbus, Ohio, US; M.R. PATEL et al.: "Antimicrobial activity of piperazine derivatives and related compounds"**

(73) Titulaire : **LABORATORIOS DEL DR. ESTEVE, S.A.**
**Av. Mare de Deu de Montserrat, 221**
**E-08026 Barcelona (ES)**

(72) Inventeur : **Colombo Pinol, Augusto**
**Av. Chile, 36, 40 1a**
**E-08032 Barcelona (ES)**
Inventeur : **Frigola Constansa, Jordi**
**Av. Diagonal, 299 at. 1a**
**E-08013 Barcelona (ES)**
Inventeur : **Pares Corominas, Juan**
**Padilla, 349 3o 3a**
**E-08025 Barcelona (ES)**
Inventeur : **Merce Vidal, Ramon**
**Marques de Sentmenat, 44 3o B**
**E-08014 Barcelona (ES)**

(74) Mandataire : **Ahner, Francis et al**
**CABINET REGIMBEAU, 26, avenue Kléber**
**F-75116 Paris (FR)**

EP 0 382 636 B1

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention se rapporte à des nouveaux dérivés de pyrimidine, 2-(4-(α-hétéroaryl-α-aryl-α-(alkyl)-méthoxy)-butyl)-1-pipérazinyl), leur procédé de préparation, ainsi que leur application en tant que médicaments.

Les composés objets de la présente invention peuvent être utilisés dans l'industrie pharmaceutique comme intermédiaires et pour la préparation de médicaments.

Il n'est connu que peu d'exemples relationnés. Dans la demande de brevet français n° 8 705 118 on décrit de nombreux éthers d'aryl-hétéroarylcarbinols, mais aucun avec pyrimidine 2-(4-butyl)-1-pipérazinyl.

Patel M.R., Bellare R.A., Deliwala Ch.V. Indian J. Exp. Biol. 1971, 9 (1), 117 et Sourlikar U.S., Shanker B., Bhide M.B. Bull. Haffkine Inst. 1977, 5(3), 90, ont décrit deux diphénylméthoxyéthers, avec la pyrimidine 2-(4-butyl)-1-pipérazinyl, en revanche il n'a pas été trouvé d'exemples avec des aryl-hétéroarylcarbinols.

Nous avons maintenant découvert que les nouveaux dérivés de pyrimidine, (2-(4-(α-hétéroaryl-α-aryl-α-alkylméthoxy)-butyl)-1-pipérazinyl) qui font l'objet de la présente invention, présentent une activité sur le système nerveux central, en particulier ils présentent une activité anxiolitique et tranquillisante, permettant leur emploi thérapeutique pour le traitement de l'anxiété.

Les composés objet de la présente invention répondent à la formule générale I.

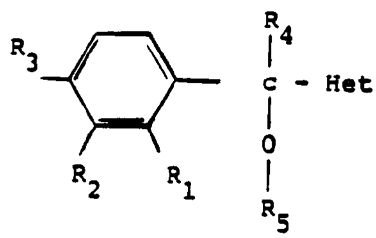

dans laquelle $R_1$, $R_2$ et $R_3$, indépendamment les uns des autres, représentent un atome d'hydrogène, un halogène, un radical alkyle inférieur de $C_1$ à $C_4$, un cycloalkyle, un radical trifluorométhyle ($CF_3$), ou un radical alcoxy.

$R_4$ représente un atome d'hydrogène, un radical alkyle inférieur de $C_1$ à $C_4$, un radical cycloalkyle, un radical alkényle inférieur, ou un radical aminoalkyle.

Het représente un azole, choisi parmi :

a) Pyrazole de formule générale

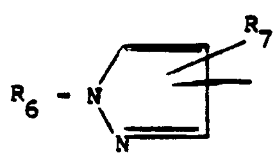

dans laquelle $R_6$ et $R_7$, indépendamment l'un de l'autre, représentent un atome d'hydrogène, un radical alkyle inférieur, un radical phényle
un radical benzyle ou un radical alkylamino
et R peut représenter, en plus, un halogène ou un radical alcoxy.

b) Imidazole de formule générale

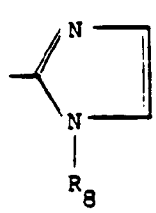

dans laquelle $R_8$ représente un atome d'hydrogène, un radical alkyle inférieur, un radical benzyle ou un radical alkylamino

Dans la formule générale I, $R_8$ représente une pyrimidinylpipérazinylalkyle, de formule générale

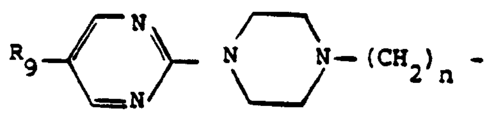

dans laquelle $R_9$ représente un atome d'hydrogène, un halogène, un alkyle inférieur, un alcoxy inférieur, ou un groupe phényle, et n peut avoir les valeurs 1 à 6.

La présente invention se rapporte également aux sels physiologiquement acceptables des composés de formule générale I.

Les dérivés de formule générale I et leurs sels sont appropriés pour prévenir ou traiter les troubles associés au récepteur sérotoninergique.

Les dérivés de formule générale I peuvent également être utilisés dans l'industrie pharmaceutique comme intermédiaires et pour la préparation de médicaments.

Les nouveaux dérivés de formule générale I peuvent être préparés, conformément à l'invention, selon l'une quelconque des méthodes suivantes.

Méthode A - Par réaction d'un composé de formule générale II

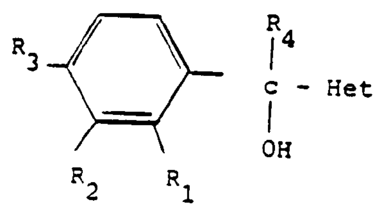

II

dans laquelle $R_1$, $R_2$, $R_3$, $R_4$ et Het ont les significations mentionnées précédemment, avec un composé de formule générale III

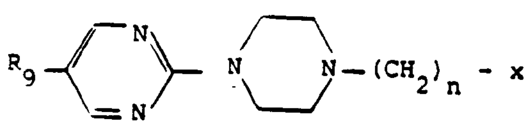

III

dans laquelle $R_9$ et n ont les significations mentionnées précédemment, et X représente un atome d'halogène ou un groupe partant choisi parmi le mésyloxy ou le tosyloxy.

La réaction d'un composé de formule générale II avec un composé de formule générale III s'effectue en présence d'un solvant adéquat, par exemple un hydrocarbure tel que le benzène ou le toluène, ou bien dans un solvant halogéné tel que le chlorure de méthylène ou le tétrachlorure de carbone, ou bien dans un éther comme le tétrahydrofuranne ou encore d'autres solvants aprotiques tels que le diméthylsulfoxyde ou la diméthylformamide. Cette réaction est avantageusement conduite en présence d'une base, telle les hydroxydes, les carbonates ou les bicarbonates des métaux alcalins, ou bien d'un mélange de ces bases.

Cette réaction est plus avantageusement conduite en présence d'un catalyseur de transfert de phase, tel que le bromure de tétrabutylammonium, ou les éthers couronnes, et dans un intervalle de température compris entre la température ambiante et la température de reflux du solvant et le temps réactionnel est compris entre 1 heure et 24 heures.

Méthode 3 - Par réaction d'un composé de formule générale IV

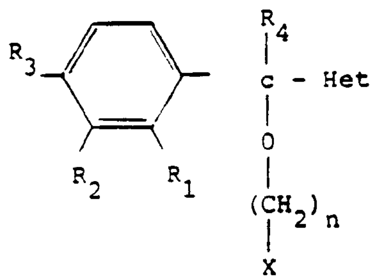

IV

dans laquelle $R_1$, $R_2$, $R_3$, $R_4$, n, X et Het ont les significations mentionnées précédemment, avec un composé de formule générale V

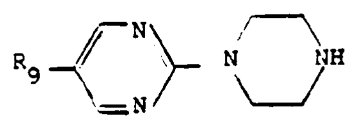

V

dans laquelle $R_9$ a les significations mentionnées précédemment.

La réaction d'un composé de formule générale IV avec un composé de formule générale V s'effectue en présence d'un solvant adéquat, par exemple une cétone, un hydrocarbure aromatique, ou un éther. Cette réaction est avantageusement conduite en présence d'une base, telle les carbonates ou bicarbonates de métaux alcalins, ou les bases organiques, telles la pyridine ou la triéthylamine. Cette réaction est plus avantageusement conduite en présence d'un catalyseur de transfert de phase, tel que le bromure de tétrabutylammonium, ou les éthers couronnes, et dans un intervalle de température compris entre la température ambiante et la température de reflux du solvant, et le temps réactionnel est compris entre 1 heure et 24 heures.

Dans les exemples suivants on indique la préparation de nouveaux dérivés selon l'invention. On décrira également quelques formes d'emploi.

Les exemples ci-après, donnés à simple titre d'illustration.

Méthode A

Exemple 1 - Préparation de Pyrimidine, 2-(4-(4-(2-(1H-imidazole-1-méthyl))-(4-chlorophényl)-méthoxy)-butyl)-1-pipérazinyl)

On chauffe à reflux pendant 18 heures un mélange de 4,6 g (15 mmoles) de 2-(1H-imidazole-1-méthyl) (4-chlorophényl)-méthanol, 4,5 g (15 mmoles) de Pyrimidine,2-(1-(4-bromobutyl)-4-pipérazine), 100 ml de soude 50 %, et 100 ml de toluène, avec 10 mg de chlorure de triéthylbenzylammonium.

La phase organique est séparée, lavée à l'eau, séchée sur du sulfate de sodium et évaporée sous vide. On obtient ainsi 3,4 g d'un liquide que l'on peut distiller à 150°C sous 0,009 bar.

Les produits des exemples 2 à 4, 6 et 7, sont obtenus selon le même mode opératoire et leurs données spectroscopiques sont présentées dans le Tableau I.

Méthode B

Exemple 5 - Préparation de Pyrimidine, 2-(4-(4-(2-(1H-imidazole-1-butyl))-(4-chlorophényl)-méthoxy)-butyl)-1-pipérazinyl).

On chauffe à reflux un mélange de 4,34 g (20 mmoles) de dichlorhydrate de pyrimidine, 2-(1-pipérazinyl), 8 g (20 mmoles) de carbonate de potassium et 6 g (20 mmoles) de 2-(1H-imidazole 1-H-butyl)(4-chlorophényl)-méthanol-O-(2-bromobutyl), dans 200 ml d'acétone. On évapore sous vide et on obtient 5,8 g de pyrimidine, 2-(4-(4-(2-(1H-imidazole-1-butyl)(4-chlorophényl)-méthoxy)-butyl)-1-pipérazinyl) sous forme liquide. Les données spectroscopiques pour son identification sont données dans le Tableau I.

TABLEAU I

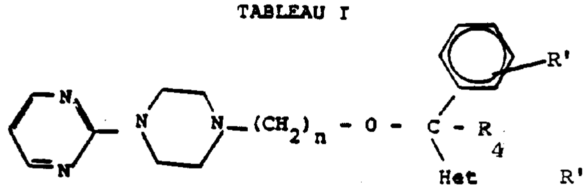

R'=$R_1$,$R_2$ ou $R_3$

| Exemple | $R_4$ | R' | Het | n | IR | 1H RMN, $Cl_3CD$, $\delta$, J=Hz |
|---|---|---|---|---|---|---|
| 1 | H | 4 Cl | 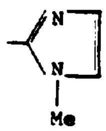 | 4 | 1590, 1490 1360, 1270 1080, 980 | 1.67(m,4H); 2.46(m,6H); 3.43 (m,5H); 3.81(m,4H); 5,66(s,1H) 6.46(t,1H,J=4.7); 6.83(s,1H); 6.97 (s,1H); 7.28(m,4H); 8.28(d,2H, J=4,7) |
| 2 | Me | 4 Cl | 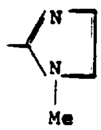 | 4 | 1590, 1550 1500, 1360 1260, 1090 980 | 1.74(m,4H); 1.99(s,3H); 2.58 (m,6H); 3.32(s,3H); 3.92(m,4H); 6.57(t,1H,J=4.7); 6.92(s,1H); 7.09(s,1H)7.3(m,4H); 8.40(d,2H, J=4.7) |
| 3 | Me | 3 $CF_3$ | 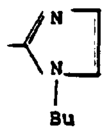 | 4 | 1590, 1460 1380, 1130 980 | 0.65(t,3H,J=6.2) 0.91(m,4H); 1.67 (m,4H); 1.94(s,3H); 2.5(m,6H); 3.7 (m,8H); 6.47(t,1H,J=4.7); 6.97 (d,2H,J=13,7); 7.5(m,4H); 8.30 (d,2H,J=4.7) |
| 4 | Me | 3 $CF_3$ | 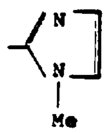 | 4 | 1590, 1490 1450, 1360 1330, 1125 980 | 1.68(m,4H); 1.90(s,3H); 2.5(m,6H); 3.25(m,5H); 3.80(m,4H); 6.48(t,1H, J=4.7); 6.95(d,2H,J=13.7); 7.4 (m,4H); 8.29(d,2H,J=4.7) |
| 5 | H | 4 Cl | 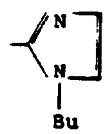 | 4 | 1590, 1500 1360, 1260 1090, 980 | 0.8(t,3H,J=6.2); 1.0-1.7(m,8H); 2.46(m,6H) 3.5(m,2H); 3.80(m,4H); 5.65(s,1H); 6.44(t,1H,J=4,7); 6.87 (s,1H); 6.98(s,1H); 7.28(s,4H), 8.27(d,2H,J=4,7) |
| 6 | H | 3 $CF_3$ | 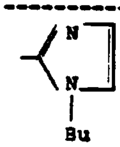 | 4 | 1580, 1450 1360, 1330 1160, 1125 1060, 970 | 0.7(t,3H,J=6.2); 1.0(m,4H); 1.65 (m,4H) 2.5(m,6H); 3.8(m,8H); 5.7 (s,1H); 6.4(t,1H,J=4.7); 6.9 (d,2H,J=13.7); 7.5(m,4H); 8.25 (d,2H,J=4.7) |
| 7 | H | 3 $CF_3$ | 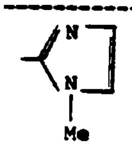 | 4 | 1590, 1500 1450, 1330 1310, 1165 1125, 980 | 1.68(m,4H); 2.4 (m,6H); 3.4(m,5H); 3.8(m,4H); 5.65(s,1H); 6.4(t,1H, J=4.7); 6.9(d,2H,J=13.7); 7.5 (m,4H); 8.3(d,2H,J=4.7) |
| 8 | H | 4 Cl | 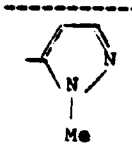 | 4 | 1585, 1485 1450, 1360 1260, 1080 980 | 1.57(m,4H); 2.38(m,6H); 3.40(m,2H) ; 3.66(m,7H), 5.35(s,1H); 5.93 (s,1H); 6.35(t,1H,J=4.7); 7.22 (s,4H); 8.20(d,2H,J=4.7) |
| 9 | Me | 4 Cl | 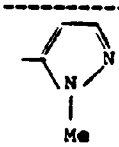 | 4 | 1585, 1480 1450, 1365 1265, 1090 980 | 1.64(m,4H); 1.76(s,3H); 2.47(m,6H) ; 3.44(s,3H); 3.82(m,4H); 6.75 (d,1H,J=1.8); 6.43(t,1H= 4.7) 7.22 (s,4H); 7.42(d,1H,J=1.8); 8.27(d,2H,J=4.7) |

Compte tenu de leurs bonnes propriétés pharmacodynamiques les dérivés de pyrimidine, 2-(4-5α-hétéroaryl-α-aryl-α-(alkyl)méthoxy)-butyl)-1-pipérazinyl), selon l'invention, peuvent être utilisés de façon satisfaisante en thérapeutique humaine et animale, en particulier dans le traitement des troubles du récepteur sérotoninergique, et plus particulièrement, pour le traitement de l'anxiété ou comme tranquillisants.

En thérapeutique humaine, la dose d'administration est bien sûr fonction de la gravité de la maladie. Elle sera généralement comprise entre environ 5 et environ 100 mg/jour. Les dérivés de l'invention seront, par exemple, administrés sous forme de comprimés, de solutions ou suspensions, ou bien de gélules.

On indiquera ci-après, à titre d'exemples, deux formes galéniques particulières des dérivés, objets de la présente invention.

Exemple de formule par comprimé

| | |
|---|---|
| Composé 2 | 5 mg |
| Lactose | 60 mg |
| Cellulose microcristalline | 25 mg |
| Povidone | 5 mg |
| Amidon prégélatiné | 3 mg |
| Dioxyde de silice colloïdale | 1 mg |
| Stéarate de magnésium | 1 mg |
| Poids comprimé | 100 mg |

Exemple de formule par gélule

| | |
|---|---|
| Composé 2 | 10 mg |
| Glycéride polyoxyéthylénée | 135 mg |
| Béhénate de glycérine | 5 mg |
| Excipient : gélatine molle q.s. | 150 mg |

Activité biologique

L'activité de certains composés sur le système nerveux central a été étudiée et plus précisément leur activité sérotoninergique, par leur union spécifique aux récepteurs 5-HT 1A.

TABLEAU II

| Exemple | Binding 5-HT-1A * déplacement (%) |
|---|---|
| 1 | 94,7 |
| 2 | 98,6 |
| 3 | 97,6 |
| 4 | 98,1 |
| 5 | 91,5 |
| 6 | 94,6 |
| 7 | 98,3 |
| 8 | 95,0 |
| 9 | 95,0 |
| Buspirone | 99,1 |
| Ipsapirone | 99,6 |

* M. HAPON : RADIOACTIVE LIGAND BINDING STUDIES : IDENTIFICATION OF CENTRAL SEROTONIN RECEPTORS dans BRAIN RECEPTOR METHODOLOGIES.

## Revendications

1. Composés hétérocycliques caractérisés en ce qu'ils répondent à la formule générale I

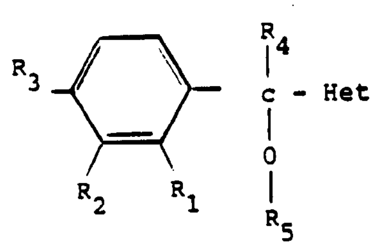

dans laquelle $R_1$, $R_2$ et $R_3$, indépendamment les uns des autres, représentent un atome d'hydrogène, un halogène, un radical alkyle inférieur de $C_1$ à $C_4$, un cycloalkyle, un radical trifluorométhyle ($CF_3$), ou un radical alcoxy.

$R_4$ représente un atome d'hydrogène, un radical alkyle inférieur de $C_1$ à $C_4$, un radical cycloalkyle, un radical alkényle inférieur, ou un radical aminoalkyle

Het représente un azole, choisi parmi :

a) Pyrazole de formule générale

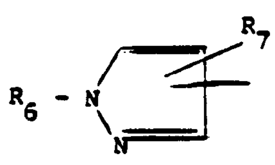

dans laquelle $R_6$ et $R_7$, indépendamment l'un de l'autre, représentent un atome d'hydrogène, un radical

alkyle inférieur, un radical phényle
un radical benzyle ou un radical alkylamino
et $R_7$ peut représenter, en plus, un halogène ou un radical alcoxy.
b)Imidazole de formule générale

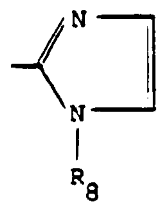

dans laquelle $R_8$ représente un atome d'hydrogène, un radical alkyle inférieur, un radical benzyle ou un radical alkylamino
$R_5$ représente une pyrimidinylpipérazinylalkyle, de formule générale

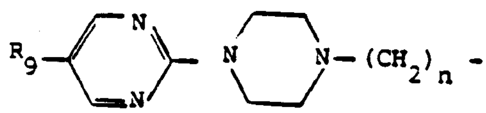

dans laquelle $R_9$ représente un atome d'hydrogène, un halogène, un alkyle inférieur, un alcoxy inférieur ou un groupe phényle et n peut avoir les valeurs 1 à 6.

**2.** Les composés répondant à la formule générale I selon la revendication 1, sélectionnés parmi le groupe suivant :
1. Pyrimidine, 2-(4-(4-(2-(1H-imidazole-1-méthyl))-(4-chlorophényl)-méthoxy)-butyl)-1-pipérazinyl)
2. Pyrimidine, 2-(4-(4-(2-(1H-Imidazole-1-méthyl))-(4-chlorophényl)(α-méthyl)-méthoxy)-butyl)-1-pipérazinyl)
3.Pyrimidine, 2-(4-(4-(2-(1H-imidazole-1-butyl))-(3-trifluorométhylphényl)(α-méthyl)-méthoxy)-butyl)-1-pipérazinyl)
4. Pyrimidine, 2-(4-(4-(2-(1H-imidazole-1-méthyl))-(3-trifluorométhylphényl)(α-méthyl)-méthoxy)-butyl)-1-pipérazinyl).

**3.** Les composés répondant à la formule générale I selon la revendication 1, sélectionnés parmi le groupe suivant :
5. Pyrimidine, 2-(4-(4-(2-(1H-imidazole-1-butyl))-(4-chlorophényl)-méthoxy)-butyl)-1-pipérazinyl)
6. Pyrimidine, 2-(4-(4-(2-(1H-imidazole-1-butyl))-(3-trifluorométhylphényl)-méthoxy)-butyl)-1-pipérazinyl)
7. Pyrimidine, 2-(4-(4-(2-(1H-imidazole-1-méthyl))-(3-trifluorométhylphényl)-méthoxy)-butyl)-1-pipérazinyl)
8. Pyrimidine, 2-(4-(4-(5-(1H-pyrazole-1-méthyl))-(4-chlorophényl)-méthoxy)-butyl)-1-pipérazinyl)
9. Pyrimidine, 2-(4-(4-(5-(1H-pyrazole-1-méthyl))-(4-chlorophényl)(α-méthyl)-méthoxy)-butyl)-1-pipérazinyl).

**4.** Procédé de préparation des composés selon l'une des revendications 1 à 3, caractérisé par la mise en oeuvre d'au moins l'une des opérations suivantes :
4.1 - Par réaction d'un composé de formule générale III

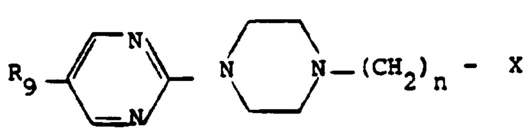
III

dans laquelle $R_9$ et X ont les significations mentionnées précédemment et n représente un entier de 1

à 6 avec un composé de formule générale II

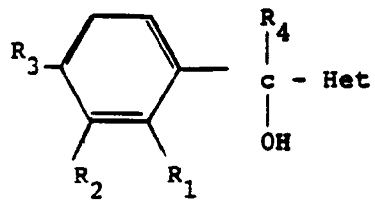

II

dans laquelle $R_1$, $R_2$, $R_3$, $R_4$ et Het ont les significations mentionnées précédemment.

4.2.- Par réaction d'un composé de formule générale IV

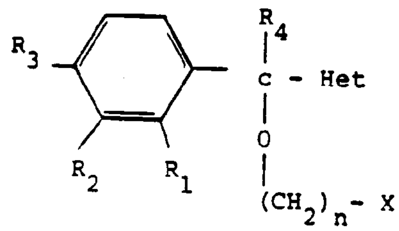

IV

dans laquelle $R_1$, $R_2$, $R_3$, $R_4$, Het, X et n ont les significations mentionnées précédemment, avec un composé de formule générale V

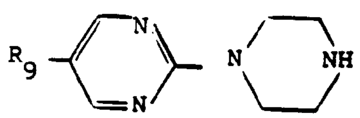

V

dans laquelle $R_9$ a les significations mentionnées précédemment.

5. A titre de médicaments, les dérivés de formule générale I et leurs sels thérapeutiquement acceptables, selon les revendications 1 à 4, en particulier à titre de médicaments destinés au traitement de certaines maladies du système nerveux central, plus particulièrement au traitement des troubles associés au récepteur sérotoninergique.

6. Compositions pharmaceutiques, caractérisées par le fait qu'elles contiennent, outre un support pharmaceutiquement acceptable, au moins un dérivé de formule générale I ou l'un de ses sels physiologiquement acceptables, selon l'une des revendications 1 à 5.

7. Utilisation des dérivés de formule générale I et de leurs sels physiologiquement acceptables, selon l'une des revendications 1 à 6, pour la fabrication de médicaments destinés au traitement de l'anxiété, en particulier pour la fabrication de tranquillisants et/ou d'anxiolytiques.

**Patentansprüche**

1. Heterocyclische Verbindungen, dadurch gekennzeichnet, daß sie der allgemeinen Formel (I) entsprechen:

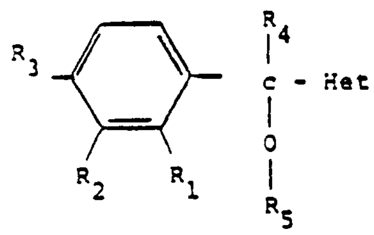

worin:

$R_1$, $R_2$ und $R_3$ unabhängig voneinander stehen für ein Wasserstoffatom, ein Halogenatom, einen niederen Alkylrest mit 1 bis 4 Kohlenstoffatomen, einen Cycloalkylrest, einen Trifluoromethylrest ($CF_3$) oder einen Alkoxyrest,

$R_4$ steht für ein Wasserstoffatom, einen niederen Alkylrest mit 1 bis 4 Kohlenstoffatomen, einen Cycloalkylrest, einen niederen Alkenylrest oder einen Aminoalkylrest,

Het steht für ein Azol, ausgewählt aus:

a) einem Pyrazol der allgemeinen Formel

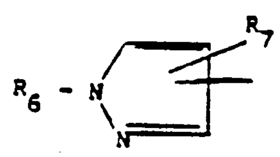

worin $R_6$ und $R_7$ unabhängig voneinander stehen für ein Wasserstoffatom, einen niederen Alkylrest, einen Phenylrest, einen Benzylrest oder einen Alkylaminorest und $R_7$ darüber hinaus für ein Halogenatom oder einen Alkoxyrest stehen kann;

b) einem Imidazol der allgemeinen Formel

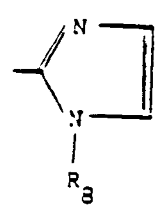

worin $R_8$ steht für ein Wasserstoffatom, einen niederen Alkylrest, einen Benzylrest oder einen Alkylaminorest, $R_5$ steht für einen Pyrimidinylpiperazinylalkylrest der allgemeinen Formel

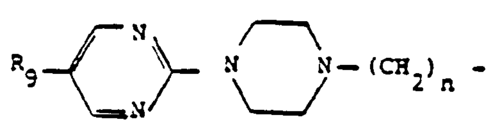

worin $R_9$ ein Wasserstoffatom, ein Halogenatom, eine niedere Alkylgruppe, eine niedere Alkoxygruppe oder eine Phenylgruppe darstellt und n die Werte 1 bis 6 haben kann.

**2.** Verbindungen der allgemeinen Formel (I) nach Anspruch 1, die ausgewählt werden aus der folgenden Gruppe:

1. 2-(4-(4-(2-(1H-Imidazol-1-methyl))-(4-chlorophenyl)-methoxy)-butyl)-1-piperazinyl)-pyrimidin
2. 2-(4-(4-(2-(1H-Imidazol-1-methyl))-(4-chlorophenyl)(α-methyl)-methoxy)-butyl)-1-piperazinyl)-pyrimidin
3. 2-(4-(4-(2-(1H-Imidazol-1-butyl))-(3-trifluoromethylphenyl)(α-methyl)methoxy)-butyl)-1-piperazinyl)-pyrimidin
4. 2-(4-(4-(2-(1H-Imidazol-1-methyl))-(3-trifluoromethylphenyl)(α-methyl)-methoxy)-butyl)-1-piperazinyl)-pyrimidin.

**3.** Verbindungen der allgemeinen Formel (I) nach Anspruch 1, die ausgewählt werden aus der folgenden Gruppe:

5. 2-(4-(4-(2-(1H-Imidazol-1-butyl))-(4-chlorophenyl)-methoxy)-butyl)-1-piperazinyl)-pyrimidin
6. 2-(4-(4-(2-(1H-Imidazol-1-butyl))-(3-trifluoromethylphenyl)-methoxy)-butyl)-1-piperazinyl)-pyrimidin
7. 2-(4-(4-(2-(1H-Imidazol-1-methyl))-(3-trifluoromethylphenyl)-methoxy)-butyl)-1-piperazinyl)-pyrimidin
8. 2-(4-(4-(5-(1H-Pyrazol-1-methyl))-(4-chlorophenyl)-methoxy)-butyl)-1-piperazinyl)-pyrimidin
9. 2-(4-(4-(5-(1H-Pyrazol-1-methyl))-4-chlorophenyl)($\alpha$-methyl)-methoxy)-butyl)-1-piperazinyl)-pyrimidin .

**4.** Verfahren zur Herstellung der Verbindungen nach einem der Ansprüche 1 bis 3, dadurch gekennzeichnet, daß mindestens eine der folgenden Operationen durchgeführt wird:

- 4.1 Umsetzung einer Verbindung der allgemeinen Formel (III)

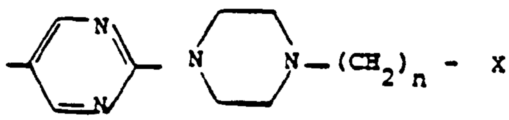

III

worin $R_9$ und X die oben angegebenen Bedeutungen haben und n eine ganze Zahl von 1 bis 6 darstellt, mit einer Verbindung der allgemeinen Formel (II)

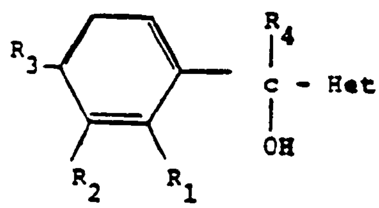

II

worin $R_1$, $R_2$, $R_3$, $R_4$ und Het die oben angegebenen Bedeutungen haben;

- 4.2 Umsetzung einer Verbindung der allgemeinen Formel (IV)

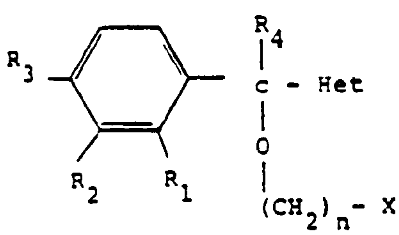

IV

worin $R_1$, $R_2$, $R_3$, $R_4$, Het, X und n die obengenannten Bedeutungen haben, mit einer Verbindung der allgemeinen Formel (V)

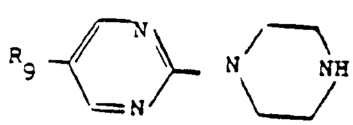

V

worin $R_9$ die obengenannten Bedeutungen hat.

**5.** Als Arzneimittel die Derivate der allgemeinen Formel (I) und ihre therapeutisch akzeptablen Salze nach den Ansprüchen 1 bis 4, insbesondere als Arzneimittel für die Behandlung von bestimmten Erkrankungen des Zentralnervensystems, ganz speziell für die Behandlung von Störungen, die in Verbindung stehen mit dem serotoninergischen Rezeptor.

6. Pharmazeutische Zusammensetzungen, dadurch gekennzeichnet, daß sie außer einem pharmazeutisch akzeptablen Träger mindestens ein Derivat der allgemeinen Formel (I) oder eines seiner physiologisch akzeptablen Salze nach einem der Ansprüche 1 bis 5 enthalten.

7. Verwendung der Derivate der allgemeinen Formel (I) und ihrer physiologisch akzeptablen Salze nach einem der Ansprüche 1 bis 6 für die Herstellung von Arzneimitteln für die Behandlung von Angstzuständen, insbesondere für die Herstellung von Tranquilizern und Anxiolytika.

## Claims

1. Heterocyclic compounds characterized in that they correspond to the general formula I

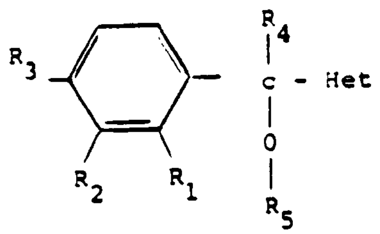

I

in which $R_1$, $R_2$ and $R_3$, independently of one another, represent a hydrogen atom, a halogen, a lower alkyl radical of $C_1$ to $C_4$, a cycloalkyl, a trifluoromethyl radical ($CF_3$), or an alkoxy radical,

$R_4$ represents a hydrogen atom, a lower alkyl radical of $C_1$ to $C_4$, a cycloalkyl radical, a lower alkenyl radical, or an aminoalkyl radical.

Het represents an azole, selected from among:

a) Pyrazole of the general formula

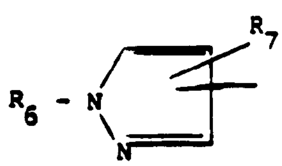

in which $R_6$ and $R_7$, independently of one another, represent a hydrogen atom, a lower alkyl radical, a phenyl radical a benzyl radical or an alkyl amino radical and $R_7$ can represent, in addition, a halogen or an alkoxy radical,

b) Imidazole of the general formula

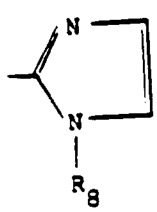

in which $R_8$ represents a hydrogen atom, a lower alkyl radical, a benzyl radical or an alkylamino radical,
$R_5$ represents pyrimidinylpiperazinyalkyl, of the general formula

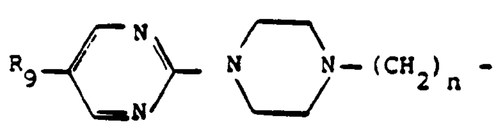

in which $R_9$ represents a hydrogen atom, a halogen, a lower alkyl, a lower alkoxy or a phenyl group,

and n may have the valuest 1 to 6.

**2.** The compounds corresponding to the general formula I according to claim 1, selected from among the following group:
1. 2-(4-(4-(2-(1H-imidazole-1-methyl))-(4-chlorophenyl)-methoxy)-butyl)-1-piperazinyl pyrimidine
2. 2-(4-(4-(2-(1H-imidazole-1-methyl))-(4-chlorophenyl)(α-methyl)-methoxy)-butyl)-1-piperazinyl)pyrimidine
3. 2-(4-(4-(2-(1H-imidazole-1-butyl))-(3-trifluoromethylphenyl)(α-methyl)-methoxy)-butyl)-1-piperazinyl) pyrimidine
4. 2-(4-(4-(2-(1H-imidazole-1-methyl))-(3-trifluoromethylphenyl)(α -methyl)-methoxy)-butyl)-1-piperazinyl) pyrimidine

**3.** Compounds corresponding to the general formula I according to claim 1, selected from among the following group:
5. 2-(4-(4-(2-(1H-imidazole-1-butyl))-(4-chlorophenyl)-methoxy)-butyl)-1-piperazinyl) pyrimidine
6. 2-(4-(4-(2-(1H-imidazole-1-butyl))-(3-trifluoromethylphenyl)-methoxy)-butyl)-1-piperazinyl) pyrimidine
7. 2-(4-(4-(2-(1H-imidazole-1-methyl))-(3-trifluoromethylphenyl)-methoxy)-butyl)-1-piperazinyl) pyrimidine
8. 2-(4-(4-(5-(1H-pyrazole-1-methyl))-(4-chlorophenyl)-methoxy)-butyl)-1-piperazinyl) pyrimidine
9. 2-(4-(4-(5-(1H-pyrazole-1-methyl))-(4-chlorophenyl)(α-methyl)-methoxy)-butyl)-1-piperazinyl) pyrimidine

**4.** Process for the preparation of the compounds according to one of claims 1 to 3, characterized by the employment of at least one of the following operations:
4.1 - By the reaction of a compound of the general formula III

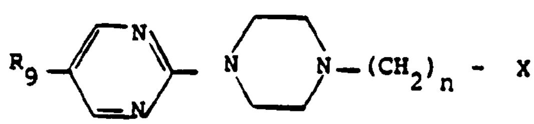

III

in which $R_9$ and X have the previously mentioned meanings and n represents an integer from 1 to 6 with a compound of the general formula II

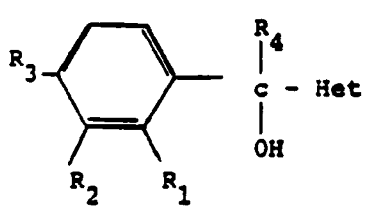

II

in which $R_1$, $R_2$, $R_3$, $R_4$ and Het have the previously mentioned meanings.
4.2 - By the reaction of a compound of the general formula IV

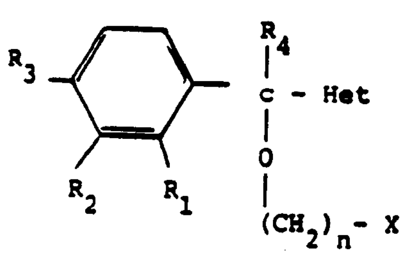

IV

in which $R_1$, $R_2$, $R_3$, $R_4$ and Het, X and n have the previously mentioned meanings, with a compound

of the general formula V

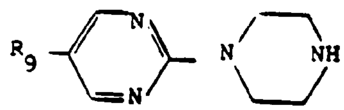

V

in which $R_9$ has the previously mentioned meanings.

5. As medicaments, derivatives of general formula I and their therapeutically acceptable salts, according to claims 1 to 4, in particular as medicaments intended for the treatment of certain disorders of the central nervous system, more particularly for the treatment of disorders associated with the serotoninergic receptor.

6. Pharmaceutical compositions, characterized by the fact that they contain, besides a pharmaceutically acceptable support, at least one derivative of the general formula I or one of its physiologically acceptable salts, according to one of claims 1 to 5.

7. Use of the derivatives of the general formula I and their physiologically acceptable salts, according to one of claims 1 to 6, for the manufacture of medicaments intended for the treatment of anxiety, in particular for the manufacture of tranquilizers and/or of anxiolytic agents.